Europäisches Patentamt

European Patent Office

Office européen des brevets

(11) Publication number: **0 306 984 B1**

(12) **EUROPEAN PATENT SPECIFICATION**

(45) Date of publication of patent specification: **15.04.92**     (51) Int. Cl.⁵: **A61K 9/08**, A61K 47/00

(21) Application number: **88114804.3**

(22) Date of filing: **09.09.88**

(54) **Preservative system for ophtalmic formulations.**

(30) Priority: **11.09.87 US 96173**

(43) Date of publication of application:
**15.03.89 Bulletin 89/11**

(45) Publication of the grant of the patent:
**15.04.92 Bulletin 92/16**

(84) Designated Contracting States:
**AT BE CH DE FR GB IT LI LU NL SE**

(56) References cited:
**WO-A-85/04106**
**DE-A- 3 026 402**
**US-A- 4 087 538**

**CHEMICAL ABSTRACTS, vol.88, no.25, 19
June 1978, Columbus, OH (US); M.T.NADIR et
al., p.166, no.183735c**

(73) Proprietor: **SYNTEX (U.S.A.) INC.
3401 Hillview Avenue
Palo Alto California 94303(US)**

(72) Inventor: **Roger Fu, Cherng-Chyi
14050 Shadow Oaks Way
Saratoga, CA 95070(US)**
Inventor: **Lidgate, Deborah M.
325 Arboleda Drive
Los Altos, CA 94022(US)**

(74) Representative: **Barz, Peter, Dr. et al
Patentanwälte Dr. V. Schmied-Kowarzik
Dipl.-Ing. G. Dannenberg Dr. P. Weinhold Dr.
D. Gudel Dipl.-Ing. S. Schubert Dr. P. Barz
Siegfriedstrasse 8
W-8000 München 40(DE)**

Rank Xerox (UK) Business Services

## Description

The present invention relates to improved ophthalmic formulations, particularly to ophthalmic formulations for anti-inflammatory drugs, and specifically to an improved preservative system for ophthalmic formulations of carboxyl ("-COOH") group-containing drugs, especially non-steroidal anti-inflammatory drugs ("NSAIDs").

The invention also relates to methods of using these formulations for treating diseases that are either caused by, associated with or accompanied by inflammatory processes, including, among others, glaucoma, cystoid macular edema, uveitis, diabetic retinopathy, and conjunctivitis, or any trauma caused by eye surgery or eye injury.

The topical use of NSAIDs, particularly pyrrolo pyrroles, in the treatment of ophthalmic diseases was first taught in U.S. Patent No. 4,454,151, where NSAID compounds (such as those described in U.S. Patents 4,089,969; 4,232,038; 4,087,539 and 4,097,579) were exemplified in formulation with $NaH_2PO_4 \cdot H_2O$, $Na_2HPO_4 \cdot H_2O$, NaCl, benzalkonium chloride ("BAC") and sterilized water. While the formulations described in the '151 patent were efficacious, an insoluble complex was found to form between the NSAID and the BAC. The formulations became cloudy or turbid and did not, therefore, have the stability desired for shelf life in commercial applications. A reasonable minimum shelf life (that is, the time during which a solution remains clear and retains its pharmaceutical activity) is at least about one year, representing sufficient time to package, ship, and store a formulation without having to replace expired stock too frequently. The solutions of the present invention have shown a shelf life of at least one year. Thus, the present invention entails an improvement over the formulations described in the '151 patent.

In general, an opthalmic formulation contains an active compound and various ophthalmologically acceptable excipients, in the form of a solution, an ointment, a suspension, etc. An excipient is ophthalmologically acceptable if it is non-irritating to the eye and if its active ingredient penetrates the blood-aqueous barrier and/or diffuses through the various ocular substructures to the site where it is pharmacologically active. The excipients can include a tonicifier, a preservative, a surfactant, a buffering system, a chelating agent, a viscosity agent as well as other stabilizing agents. Ophthalmic formulations must be sterile, and if intended for multiple dosing regimens, must be preserved with an effective anti-microbial agent.

Organo-mercurials (e.g., thimerosal, phenylmercuric acetate and phenylmercuric nitrate) have been used extensively as the preservative in ophthalmic solutions. These compounds, however, pose difficulties due to potential mercury toxicity as well as poor chemical stability. Benzalkonium chloride, a quaternary ammonium compound, has been widely used in ophthalmic solutions, and is considered to be the preservative of choice. However, BAC has typically been considered to be incompatible with anionic drugs (e.g., salicylates or nitrates, etc.), forming insoluble complexes which cause the solution to become cloudy or turbid. Such a complex between the anionic drug and benzalkonium chloride can cause a decrease in the pharmaceutical activity of the anionic drug.

Many NSAIDs (such as ketorolac, indomethacin, flurbiprofen and diclofenac) are being developed for ocular use because of their activity as anti-inflammatory agents including their ability to prevent cystoid macular edema.

In the past, as in the case with other ophthalmic drugs that contain a -COOH group, antiinflammatory solutions of NSAIDs for occular use have proven to be incompatible with quaternary ammonium compounds such as BAC. This incompatibility is due to the fact that the -COOH group can form a complex with the quaternary ammonium compounds, rendering the preservative less available to serve its function, and reducing the activity of the active ingredient. Indomethacin ophthalmic formulations have been prepared, however, these are suspensions, not solutions. Ocufen Ophthalmic solution, an NSAID (flurbiprofen) approved by the FDA for ophthalmic use, incorporates thimerosal (with EDTA) as its preservative system. In U.S. patent 4,454,151 there is a disclosure of an ophthalmic formulation using ketorolac, benzalkonium chloride (as the preservative) and polysorbate 80, however the solution became cloudy or turbid after a short period of time.

It has remained desired to provide a stable, clear, antimicrobially effective ophthalmic formulation with a prolonged shelf life for -COOH group containing ophthalmic drugs, especially NSAIDs, using BAC as the preservative.

It has now been discovered that stable, clear and antimicrobially effective, NSAID-containing ophthalmic formulations can be prepared which include BAC. These solutions have an improved shelf life, exhibiting no cloudiness or turbidity over extended periods.

In one aspect of the invention, these compositions include an ophthalmologically effective amount of a NSAID, BAC (preservative) and a stabilizing amount of Octoxynol 40 (nonionic surfactant), all in an aqueous vehicle.

Another aspect is an ophthalmic composition including an ophthalmologically effective amount of ketorolac or an isomer, an ester, or a pharmaceutically acceptable salt thereof, benzalkonium chloride as a preservative and a stabilizing amount of Octoxynol 40 as a nonionic surfactant.

The ophthalmic pharmaceutical formulations of the invention can be used for treating ophthalmic diseases in mammals. These diseases are those that are either caused by, associated with or accompanied by inflammatory processes, including, among others, glaucoma, cystoid macular edema, uveitis, diabetic retinopathy and conjunctivitis, or any trauma caused by eye surgery or eye injury.

Definitions

As used herein, the term "NSAID" means an ophthalmologically acceptable non-steroidal anti-inflammatory drug. The NSAID's include, for example, flurbiprofen, ketorolac, diclofenac, indomethacin, and the isomers, esters, and pharmaceutically acceptable salts thereof.

As used herein, the term "q.s." means adding a quantity sufficient to achieve a stated function, e.g., to bring a solution to the desired volume (i.e., 100%).

As used herein, the term "treatment" or "treating" means any treatment of a disease in a mammal, including:

(i) preventing the disease, that is, causing the clinical symptoms of the disease not to develop;

(ii) inhibiting the disease, that is, arresting the development of clinical symptoms; and/or

(iii) relieving the disease, that is, causing the regression of clinical symptoms.

As used herein, the term "effective amount" means a dosage sufficient to provide treatment for the disease state being treated. This will vary depending on the patient, the disease and the treatment being effected.

As used herein, the term "antimicrobially effective" means ability to withstand the U.S. Pharmacopia antimicrobial challenge.

As used herein, the term "stabilizing" means keeping a formulation clear and antimicrobially effective for its minimum reasonable shelf life, e.g., at least one year.

Formulations

The formulations of the present invention include an NSAID active agent in an effective amount for ophthalmic treatment, beuzalkonium chloride, a stabilizing amount of Octoxynol 40, optionally including other excipients such as a chelating agent, a tonicifier, a buffering system, a viscosity agent as well as other stabilizing agents. Ophthalmic solutions and suspensions typically contain an aqueous vehicle rather than an oily vehicle. Ophthalmic formulations must be sterile, and if intended for multiple dosing regimens, must be antimicrobially effective for their minimum reasonable shelf life, e.g., at least one year, and preferably two to three years or more. The ingredients used in the formulations of the present invention are typically commercially available or can be made by methods readily known to those skilled in the art.

Pharmaceutical ophthalmic formulations typically contain an effective amount, e.g., 0.001% to 10% wt/vol., preferably 0.002% to 5% wt/vol, most preferably 0.005% to 1% wt/vol of an active ingredient (e.g., the NSAID of the present invention). The amount of active ingredient will vary with the particular formulation and the disease state for which it is intended. The total concentration of solutes should be such that, if possible, the resulting solution is isotonic with the lacrimal fluid (though this is not absolutely necessary) and has a pH in the range of 6 to 8.

The formulations of the present invention are prepared as solutions incorporating the above-described ingredients within the following approximate ranges:

| Ingredient | Amount |
| --- | --- |
| Active Agent | 0.001% to 10.0% wt/vol.; |
| BAC | 0.001% to 1.0% wt/vol.; |
| Octoxynol 40 | 0.001% to 1.0% wt/vol.; |
| Other Excipients | 0% to 10.0% wt/vol.; and |
| Purified Water | q.s. to 100%. |

Optional other excipients, such as a chelating agent and a tonicifier, are used in the following approximate proportions:

| Ingredient | Amount |
|---|---|
| Chelating agent | 0.01% to 1.0%wt/vol.; |
| Tonicifier | q.s. to achieve isotonicity with lacrimal fluid; and |
| 1N NaOH or 1N HCl | q.s. to adjust pH to 6.0 to 8.0. |

In a preferred ophthalmic NSAID solution, the ingredients are combined in the following proportions:

| Ingredient | Amount |
|---|---|
| NSAID | 0.002% to 5.0% wt/vol.; |
| BAC (50% aq. soln.) | 0.002% to 1.0% wt/vol.; |
| Octoxynol 40 (70% aq. soln.) | 0.001% to 1.0% wt/vol.; |
| EDTA Na$_2$ | 0.01% to 1.0% wt/vol.; |
| NaCl | q.s. for isotonicity with lacrimal fluid; |
| 1N NaOH or 1N HCl | q.s. to adjust pH to 7.4±0.4; and |
| Purified Water | q.s. to 100%. |

In another preferred ophthalmic NSAID solution, the ingredients are combined in the following proportions:

| Ingredient | Amount |
|---|---|
| NSAID | 0.005% to 1.0% wt/vol.; |
| BAC (50% aq. soln.) | 0.002% to 1.0% wt/vol.; |
| Octoxynol 40 (70% aq. soln.) | 0.001% to 1.0% wt/vol.; |
| EDTA Na$_2$ | 0.01% to 1.0% wt/vol.; |
| NaCl | q.s. for isotonicity with lacrimal fluid; |
| 1N NaOH or 1N HCl | q.s. to adjust pH to 7.4±0.4; and |
| Purified Water | q.s. to 100%. |

In a more preferred ophthalmic NSAID solution, the ingredients are combined in the following proportions:

| Ingredient | Amount |
|---|---|
| NSAID | 0.50% wt/vol.; |
| BAC (50% aq. soln.) | 0.02% wt/vol.; |
| Octoxynol 40 (70% aq. soln.) | 0.01% wt/vol.; |
| EDTA Na$_2$ | 0.10% wt/vol.; |
| NaCl | q.s. for isotonicity with lacrimal fluid; |
| 1N NaOH or 1N HCl | q.s. to adjust pH to 7.4±0.4; and |
| Purified Water | q.s. to 100%. |

The invention relates primarily to formulations having as the active agent ophthalmologically acceptable drugs (including the isomers, esters and pharmaceutically acceptable salts thereof) that can form a complex with BAC, particularly NSAIDs and drugs with a carboxyl group.

NSAIDs useful in the practice of this invention include, for example, ketorolac (and the other compounds described as being ophthalmologically effective in U.S. Patent No. 4,454,151 to Waterbury, issued June 12, 1984, the pertinent portions of which are incorporated herein by reference), indomethacin, flurbiprofen sodium, and diclofenac, including the isomers, esters and pharmaceutically acceptable salts thereof.

The nonionic surfactant used in the formulations of the present invention, (i.e., Octoxynol 40) is a octylphenoxypoly(ethyleneoxy)ethanol, the mole ratio of ethylene oxide to octylphenol being 40. Octoxynol 40 is manufactured and sold by GAF under the trade name Igepal® CA897 (a 70% aqueous solution of Octoxynol 40).

Among the optional excipients, the chelating agents useful in the formulations of the present invention

4

include 8-hydroxyquinoline sulfate, citric acid, and preferably disodium edetate. Under certain conditions, the chelating agent may also enhance the anti-microbial effect due to its ability to render essential metal ions unavailable to the microbes.

Buffering systems optionally useful in the formulations of the present invention are based on, for example, citrate, borate, or phosphate.

Tonicifiers optionally useful in the formulations of the present invention include dextrose, potassium chloride and/or sodium chloride, preferably sodium chloride.

Viscosity agents optionally useful in the formulations of the present invention include the cellulose derivatives such as hydroxypropylmethyl cellulose, sodium carboxymethylcellulose, and hydroxyethylcellulose.

Other optional excipients useful in the formulations of the present invention include stabilizing agents such as antioxidants, e.g., sodium metabisulfate and ascorbic acid, depending on the NSAID used.

These formulations are prepared by dissolving the solutes (e.g., the NSAID, BAC, the Octoxynol 40, the chelating agent, and the buffering agent) in a suitable quantity of water, adjusting the pH to about 6 to 8, preferably 6.8 to 8.0 and most preferably 7.4, making a final volume adjustment to 100% with additional water, and sterilizing the preparation using any suitable method known to those in the art.

It has been discovered that ophthalmic formulations incorporating the preservative system of the invention are physically stable (i.e., remain clear) and functionally stable (i.e., remain antimicrobially effective) for at least the minimum reasonable shelf life of such products.

Preferred Formulations

The preferred chelating agent of the invention is disodium edetate.

The preferred ophthalmic solutions of the invention include a NSAID, benzalkonium chloride, Octoxynol 40 and disodium edetate.

In a preferred ophthalmic NSAID solution, the ingredients are combined in the following proportions:

| Ingredient | Amount |
|---|---|
| NSAID | 0.002% to 5.0% wt/vol.; |
| BAC (50% aq. soln.) | 0.002% to 1.0% wt/vol.; |
| Octoxynol 40 (70% aq. soln.) | 0.001% to 1.0% wt/vol.; |
| EDTA Na$_2$ | 0.01% to 1.0% wt/vol.; |
| NaCl | q.s. for isotonicity with lacrimal fluid; |
| 1N NaOH or 1N HCl | q.s. to adjust pH to 7.4±0.4; and |
| Purified Water | q.s. to 100%. |

In another preferred ophthalmic NSAID solution, the ingredients are combined in the following proportions:

| Ingredient | Amount |
|---|---|
| NSAID | 0.005% to 1.0% wt/vol.; |
| BAC (50% aq. soln.) | 0.002% to 1.0% wt/vol.; |
| Octoxynol 40 (70% aq. soln.) | 0.001% to 1.0% wt/vol.; |
| EDTA Na$_2$ | 0.01% to 1.0% wt/vol.; |
| NaCl | q.s. for isotonicity with lacrimal fluid; |
| 1N NaOH or 1N HCl | q.s. to adjust pH to 7.4±0.4; and |
| Purified Water | q.s. to 100%. |

A preferred ophthalmic NSAID solution has the following formulation:

| Ingredient | Amount |
|---|---|
| NSAID | 0.50% wt/vol. |
| BAC (50% aq. soln.) | 0.02% wt/vol. |
| Octoxynol 40 (70% aq. soln.) | 0.01% wt/vol. |
| EDTA Na$_2$ | 0.10% wt/vol. |
| NaCl | q.s. for isotonicity with lacrimal fluid |
| 1N NaOH or 1N HCl | q.s. to adjust pH to 7.4±0.4 |
| Purified Water | q.s. to 100% |

Most preferred is the ophthalmic solution according to the above formulation wherein the NSAID is Ketorolac Tromethamine or an isomer thereof.

Utility and Administration

This invention is directed to NSAID ophthalmic formulations useful for treating ophthalmic diseases in mammals. These diseases are either caused by, associated with or accompanied by inflammatory processes, including, among others, glaucoma, cystoid macular edema, uveitis, diabetic retinopathy and conjunctivitis, or any trauma caused by eye surgery or eye injury.

The treatment is both curative and preventative. Where applied, for example, pre-surgically or immediately post-traumatically, i.e. before inflammation develops, it prevents develpment of inflammation. When applied directly to the eye suffering from any of the named ophthalmic diseases, it suppresses already developed inflammatory processes.

Ophthalmic formulations are typically administered by topical application to the eyelids or for instillation into the space (cul-de-sac) between the eyeball and the eyelids, of topically applied ophthalmic solutions, suspensions or ointments, or by subconjunctival injection.

The dosage level will, of course, depend on the concentration of the drops, the condition of the subject and the individual magnitude of responses to treatment. However, typical dosage ranges might be about 2 to 10 drops of 0.5% solution of active ingredient per day.

For a more detailed discussion of ophthalmic formulations, their preparation and administration, see Remington's Pharmaceutical Sciences, 15th Ed., pages 1489-1504, (1975).

Testing

Ophthalmic formulations such as the solutions of the present invention are typically tested for physical stability, chemical stability, and preservative efficacy, both when they are first manufactured and after a fixed period of time (e.g., after two years). They are generally considered to be safe and clinically acceptable if proven to be well tolerated in the eye.

Physical stability is determined by observation of a solution after expiration of a fixed period of time. A solution is considered to be physically stable if its appearance (e.g., color and clarity) does not change and if the pH remain constant, within acceptable limits. Chemical stability involves a routine chemical analysis of the solution, to be sure that its active ingredient and the excipients have not changed after a fixed period of time.

Preservative efficacy is tested by the procedure described in the U.S. Pharmacopia Compendiary, whereby a solution is challenged with a microbe and a determination is made as to whether the microbe survives in it.

The following examples are given to enable those skilled in the art to more clearly understand and to practice the present invention. They should not be considered as a limitation on the scope of the invention, but merely as being illustrative and representative thereof.

EXAMPLE 1

This example illustrates the preparation of a representative pharmaceutical formulation for ophthalmic administration containing the NSAID Ketorolac Tromethamine.

| Ingredient | Amount |
|---|---|
| Ketorolac Tromethamine | 0.50% wt/vol. |
| BAC (50% aq. soln.) | 0.02% wt/vol. |
| Octoxynol 40 (70% aq. soln.) | 0.01% wt/vol. |
| EDTA Na$_2$ | 0.10% wt/vol. |
| NaCl | 0.79% wt/vol. |

The above ingredients are mixed, adding purified water until they are dissolved, the pH is adjusted to 7.4±0.4 and the balance of the formulation is made up with purified water, adding a quantity sufficient to make 100% volume. The solution is then sterilized.

Other NSAIDs or their isomers, salts or esters, such as those described above, can be used as the active compound in the preparation of the formulation of this example.

EXAMPLE 2

This example illustrates the preparation of a representative pharmaceutical formulation for ophthalmic administration containing the NSAID Ketorolac Tromethamine.

| Ingredient | Amount |
|---|---|
| Ketorolac Tromethamine | 0.50% wt/vol. |
| BAC (50% aq. soln.) | 0.02% wt/vol. |
| Octoxynol 40 (70% aq. soln.) | 0.02% wt/vol. |
| EDTA Na$_2$ | 0.20% wt/vol. |
| NaCl | 0.79% wt/vol. |

The above ingredients are mixed, adding purified water until they are dissolved, the pH is adjusted to 7.4±0.4 and the balance of the formulation is made up with purified water, adding a quantity sufficient to make 100% volume. The solution is then sterilized.

Other NSAIDs or their isomers, salts or esters, such as those described above, can be used as the active compound in the preparation of the formulation of this example.

EXAMPLE 3

This example illustrates the preparation of a representative pharmaceutical formulation for ophthalmic administration containing the NSAID Ketorolac Tromethamine.

| Ingredient | Amount |
|---|---|
| Ketorolac Tromethamine | 0.10% wt/vol. |
| BAC (50% aq. soln.) | 0.004% wt/vol. |
| Octoxynol 40 (70% aq. soln.) | 0.004% wt/vol. |
| EDTA Na$_2$ | 0.05% wt/vol. |
| NaCl | 0.88% wt/vol. |

The above ingredients are mixed, adding purified water until they are dissolved, the pH is adjusted to 7.4±0.4 and the balance of the formulation is made up with purified water, adding a quantity sufficient to make 100% volume. The solution is then sterilized.

Other NSAIDs their isomers, salts or esters, such as those described above, can be used as the active compound in the preparation of the formulation of this example.

EXAMPLE 4

This example illustrates the preparation of a representative pharmaceutical formulation for ophthalmic administration containing the NSAID flurbiprofen sodium.

| Ingredient | Amount |
|---|---|
| Flurbiprofen Sodium | 0.03% wt/vol. |
| BAC (50% aq. soln.) | 0.02% wt/vol. |
| Octoxynol 40 (70% aq. soln.) | 0.01% wt/vol. |
| EDTA Na$_2$ | 0.10% wt/vol. |
| NaCl | 0.90% wt/vol. |

The above ingredients are mixed, adding purified water until they are dissolved, the pH is adjusted to 7.4±0.4 and the balance of the formulation is made up with purified water, adding a quantity sufficient to make 100% volume. The solution is then sterilized.

Other ophthalmic drugs and NSAIDs, such as those described above, can be used as the active compound in the preparation of the formulation of this example.

EXAMPLE 5

Physical stability of the formulations of the present invention is measured by preparing clear formulations, in the concentrations shown in the table below, sealing them in sterilized containers, and observing the clarity of the solution after a period of one month and again after five months. Solutions that remain clear are considered stable in this procedure.

The formulations of the present invention have proven to be stable when tested in accordance with the above procedure. Formulations using surfactants other than the nonionic surfactant of the invention did not remain clear and were not stable.

Three surfactants were evaluated for their ability to dissolve the ketorolac - benzalkonium chloride complex and maintain a physically clear solution over an extended period of time. The three surfactants tested were: Octoxynol 40; Polysorbate 80 (Tween 80); and Myrj 52. Two concentrations of each surfactant were incorporated into the ophthalmic formulation, and these were placed at various temperatures for future visual observations.

| | Octoxynol 40 | | Tween 80 | | Myrj 52 | |
|---|---|---|---|---|---|---|
| | 0.004% | 0.02% | 0.0035% | 0.01% | 0.0015% | 0.01% |
| 1 month | | | | | | |
| 60°C | clear | clear | clear | clear | clear | clear |
| 40°C | clear | clear | very turbid | very turbid | turbid | turbid |
| RT | clear | clear | turbid | turbid | clear | clear |
| 4-40°C | clear | clear | turbid | turbid | clear | clear |
| 5 month | | | | | | |
| 60°C | clear | clear | clear | clear | clear | clear |
| 40°C | clear | clear | turbid | turbid | turbid | turbid |
| RT | clear | clear | turbid | turbid | turbid | turbid |

At the 5 month time period it was apparent that the Octoxynol 40 surfactant was superior to the other two surfactants. At 5 months, Tween 80 and Myrj 52 displayed turbidity when stored at RT. The presence of turbidity suggested the inability to solubilize a precipitate formation between the Ketorolac moiety and benzalkonium chloride.

A further study has shown a 2 year shelf life for the ophthalmic formulation. Precipitate formation and turbidity are not a problem with this formulation. Preservative efficacy is maintained throughout the 2 year shelf life.

EXAMPLE 6

Preservative efficacy of the formulations of the present invention is measured by preparing formulations, e.g., according to the foregoing Examples, and subjecting them to the U.S. Pharmacopia antimicrobial challenge.

The formulations of the present invention demonstrate preservative efficacy when tested in accordance with the above procedure.

EXAMPLE 7

The objective of this clinical efficacy study was to compare the effectiveness and safety of ketorolac with a control solution in reducing inflammation following cataract removal and intraocular lens implantation. All patients underwent an extracapsular cataract extraction with intraocular lens implantation 1 day following initiation of treatment.

Ophthalmic examinations were performed preoperatively (within 3 weeks of surgery) and during the first week (postoperative days 1 to 3), second week (postoperative days 4 through 12), and third week (postoperative days 15 through 27) or treatment. Particular attention was given to signs and symptoms consistent with inflammation. Among the ocular characteristics assessed on a scale of none, mild, moderate, or severe were: lid edema, corneal edema, conjunctival injections, ciliary flush, and the presence of cells and flare in the anterior chamber.

Fluorophotometry: Anterior segment inflammation (i.e., iritis, cyclitis, iridocyclitis) is by definition a disruption of the blood-aqueous barrier. When inflammation is present, a careful slit lamp examination will reveal cells and flare within the anterior chamber of the eye. The clinical grading of cells and flare is a measure of degree of anterior segment inflammation; but consistent grading of these observations is difficult, even by experts.

Ocular fluorophotometry is based on the fact that the blood-aqueous barrier becomes permeable to intravascular cells and proteinaceous fluid (explaining the observed cells and flare) and also to intravascular fluorescein. Furthermore, the appearance of fluorescein within the anterior chamber is a more sensitive indication of the breakdown of the blood-aqueous barrier than the gross observation of cells and flare, and is consistently quantifiable. For these reasons, a Flurortron® Master (Coherent, Sunnyvale, California), complete with software modifications designed for this study was used. Following oral administration of fluorescein, the fluorophotometer was used to determine the integrity of the aqueous barrier by measuring the concentration of fluorescein in the anterior chamber.

The fluorophotometry data were analyzed using the Wilcoxon Rank Sum Test or analysis of variance (ANOVA) of rank-transformed data by calculating the percentage difference in fluorescein concentration between the patient's two eyes, according to the formula:

Percent difference = [(fluorescein concentration of operated eye - fluorescein concentration of unoperated eye)/fluorescein concentration of unoperated eye] x 100.

This calculation allowed and corrected for any interpatient variation in the timing and concentration of fluorescein administered.

129 patients began treatment for 21 days with either ketorolac or vehicle. In this study, the ketorolac formulation used was that illustrated in Example 1 above. During the first week 118 patients and during the second week 110 patients were evaluated for postoperative inflammation with ophthalmic examinations and fluorophotometry. During the third week, 83 patients were evaluated with ophthalmic examinations alone. At 2 weeks ketorolac provide significantly greater anti-inflammatory activity than the vehicle as measured by fluorophotometry ($p = 0.019$). When patients were excluded who had greater than 40% difference in fluorescein concentration between eyes at baseline, the p-value during week 2 rose to 0.06. In addition, the vehicle-treated patients had more ocular inflammation seen on slit lamp examination, e.g., eyelid edema ($p = 0.001$), conjunctival injection ($p = 0.001$), and Descemet folds ($p = 0.002$) than did the ketorolac-treated patients. Finally, there were significantly more complaints ($p = 0.01$) and more sever complaints consistent with ocular inflammation (photophobia, iritis, conjunctival injection) in the vehicle-treated group than in the ketorolac-treated group.

In summary, ketorolac solutions proved significantly superior to vehicle in treating postoperative inflammation as quantitated by fluorophotometry, by routine slit lamp examination, by patients having fewer and milder adverse events, and by infrequent need of additional corticosteroid therapy to control inflammation.

EXAMPLE 8

This was a double-blind, parallel comparison with vehicle to evaluate the efficacy of ketorolac 0.5% ophthalmic solution in reducing signs and symptoms of allergic conjunctivitis. Ketorolac 0.5% solution or a vehicle solution of the same pH and tonicity were instilled four times daily into the eyes of patients with allergic conjunctivitis (ocular itching with and without eosinophils seen in conjunctival scrapings) for 7 days.

Thirty patients with allergic conjunctivitis participated in the study. Following admission to the study, patients reported to the investigator for baseline, mid-week, and final one-week examinations. At each of these visits, patients received ophthalmic examinations (visual acuity, external eye exam using slit lamp biomicroscopy, measurement of intraocular pressure, and undilated ophthalmoscopic examination). Laboratory tests included a conjunctival scraping performed at baseline and the final exam.

All patients completed the study. There were no adverse events or toxicities in patients treated with vehicle while stinging on one occasion was reported from ketorolac 0.5% ophthalmic solution. Ketorolac treatment was associated with a decrease in free eosinophilic granules as compared to vehicle ($p = 0.025$ Fisher's Exact Test. two-tailed).

The results of this study show that ketorolac 0.5% ophthalmic solution applied four times daily for seven days produces a decrease in eosinophilic granules as compared to vehicle in the treatment of allergic conjunctivitis.

EXAMPLE 9

This study was a double-blind, paired comparison design travel to evaluate the tolerance of ketorolac 0.5% ophthalmic solution and its vehicle in 26 healthy subjects. Solutions were instilled three times daily for 21 days. Complete ophthalmic examinations were done pretreatment and on days 3, 10, 17, 24 (2 days after ending treatment), and 45 (23 days after ending treatment). No statistically significant difference in symptoms (burning, stinging, itchiness, scratchiness, photophobia) or signs (tearing, ocular discharge, conjunctival vasodilation, chemosis, keratitis, fluorescein staining, Rose Bengal staining) was found between ketorolac and vehicle.

EXAMPLE 10

An ocular formulation containing 5 mg/ml ketorolac tromethamine was administered at a dose of 0.1 ml/eye every one-half hour for a total of 12 doses to both eyes of 6 New Zealand albino rabbits. The formulation contained benzalkonium chloride as the preservative system. Two additional groups of animals served as saline and vehicle controls, respectively.

Eyes were examined after the last dose was administered and on days 1, 2, 3, and 6 following dosing. Results indicated that no eye irritation or toxicity resulted from ketorolac tromethamine administration.

**Claims**

1. A liquid ophthalmic NSAID formulation comprising a NSAID in an effective amount for ophthalmic treatment, benzalkonium chloride, a stabilizing amount of Octoxynol 40, and an aqueous vehicle.

2. The ophthalmic NSAID formulation of Claim 1 including disodium edetate.

3. The ophthalmic NSAID formulation of any one of Claims 1 and 2 wherein said NSAID is selected from ketorolac, indomethacin, flurbiprofen, and diclofenac, or their isomers, pharmaceutically acceptable salts, or esters.

4. The ophthalmic NSAID formulation of any one of Claims 1 to 3 wherein said NSAID is Ketorolac Tromethamine.

5. The ophthalmic NSAID formulation of any one of Claims 1 to 3 wherein said NSAID is the (l)-isomer of ketorolac or one of its pharmaceutically acceptable salts.

6. The ophthalmic NSAID formulation of any one of Claims 1 to 5 comprising:

| NSAID | 0.001% to 10.0% wt/vol.; |
|---|---|
| Benzalkonium Chloride | 0.001% to 1.0% wt/vol.; |
| Octoxynol 40 | 0.001% to 1.0% wt/vol.; and |
| Purified Water | q.s. to 100%. |

**7.** The ophthalmic NSAID formulation of Claim 6 including:

| Chelating agent | 0.01% to 1.0%wt/vol.; |
|---|---|
| Tonicifier | q.s. to achieve isotonicity with lacrimal fluid; and |
| 1N NaOH or 1N HCl | q.s. to adjust pH to 6.0 to 8.0. |

**8.** The ophthalmic NSAID formulation of Claim 7 comprising:

| NSAID | 0.50% wt/vol.; |
|---|---|
| Benzalkonium Chloride (50% aq. soln.) | 0.02% wt/vol.; |
| Octoxynol 40 (70% aq. soln.) | 0.01% wt/vol.; |
| EDTA Na$_2$ | 0.10% wt/vol.; |
| NaCl | 0.79% wt/vol.; |
| 1N NaOH or 1N HCl | q.s. to adjust pH to 7.4±0.4; and |
| Purified Water | q.s. to 100%. |

**9.** The ophthalmic NSAID formulation of Claim 8 wherein said NSAID is Ketorolac Tromethamine.

**10.** An antimicrobially effective preservative system for ophthalmologically acceptable, carboxyl group-containing drugs, said preservative system comprising benzalkonium chloride and a stabilizing amount of Octoxynol 40.

**11.** The use of a formulation of any one of Claims 1 to 9 for the manufacture of a medicament for the treatment or prevention of ophthalmic diseases, particularly ocular inflammatory diseases.

**12.** The use of the preservative system of Claim 10 for the manufacture of a medicament for the treatment or prevention of ophthalmic diseases, particularly ocular inflammatory diseases.

**13.** A process for the preparation of a liquid ophthalmic NSAID formulation which comprises mixing
0.001% to 10.0% wt/vol. of an NSAID,
0.001% to 1.0% wt/vol. of benzalkonium chloride,
0.001% to 1.0% wt/vol. of Octoxynol 40,
and
Purified Water q.s. to 100%.

**14.** The process of Claim 13 which further comprises mixing
0.01% to 1.0%wt/vol. of a chelating agent,
q.s. of a tonicifier to achieve isotonicity with lacrimal fluid, and
q.s. of 1N NaOH or 1N HCl to adjust pH to 6.0 to 8.0.

**Revendications**

**1.** Formulation ophtalmique liquide de médicament anti-inflammatoire non stéroidien (MAINS), comprenant un MAINS en une quantité efficace pour un traitement ophtalmique, du chlorure de benzalkonium, une quantité stabilisante d'Octoxynol 40 et un véhicule aqueux.

**2.** Formulation ophtalmique de MAINS suivant la revendication 1, comprenant de l'édétate disodique.

11

**3.** Formulation ophtalmique de MAINS suivant l'une quelconque des revendications 1 et 2, dans laquelle ledit MAINS est choisi entre le kétorolac, l'indométhacine, le flurbiprofène et le diclofénac, ou leurs isomères, sels ou esters pharmaceutiquement acceptables.

**4.** Formulation ophtalmique de MAINS suivant l'une quelconque des revendications 1 à 3, dans laquelle ledit MAINS consiste en kétorolac-trométhamine.

**5.** Formulation ophtalmique de MAINS suivant l'une quelconque des revendications 1 à 3, dans laquelle ledit MAINS est l'isomère (l) du kétorolac ou bien l'un de ses sels pharmaceutiquement acceptables.

**6.** Formulation ophtalmique de MAINS suivant l'une quelconque des revendications 1 à 5, comprenant :

| | |
|---|---|
| MAINS | 0,001% à 10,0% en poids/volume ; |
| Chlorure de benzalkonium | 0,001% à 1,0% en poids/volume ; |
| Octoxynol 40 et | 0,001% à 1,0% en poids/volume ; |
| Eau purifiée | q.s. pour 100%. |

**7.** Formulation ophtalmique de MAINS suivant la revendication 6, comprenant :

| | |
|---|---|
| Agent chélatant | 0,01% à 1,0% en poids/volume ; |
| Agent accroissant la tonicité | q.s. pour parvenir à l'isotonicité avec le liquide lacrymal ; et |
| NaOH 1N ou HCl 1N | q.s pour l'ajustement du pH à une valeur de 6,0 à 8,0. |

**8.** Formulation ophtalmique de MAINS suivant la revendication 7, comprenant :

| | |
|---|---|
| MAINS | 0,50% en poids/volume ; |
| Chlorure de benzalkonium (solution aqueuse à 50%) | 0,02% en poids/volume ; |
| Octoxynol 40 (solution aqueuse à 70%) | 0,01% en poids/volume ; |
| EDTA Na$_2$ | 0,10% en poids/volume ; |
| NaCl | 0,79% en poids/volume ; |
| NaOH 1N ou HCl 1H | q.s pour l'ajustement du pH à une valeur de 7,4 ± 0,4 ; et |
| Eau purifiée | q.s. pour 100% |

**9.** Formulation ophtalmique de MAINS suivant la revendication 8, dans laquelle ledit MAINS consiste en kétorolac-trométhamine.

**10.** Composition de conservateur, à action bactéricide, destinée à des médicaments contenant des groupes carboxyle, acceptables en ophtalmologie, ladite composition de conservateur comprenant du chlorure de benzalkonium et une quantité stabilisante d'Octoxynol 40.

**11.** Utilisation d'une formulation suivant l'une quelconque des revendications 1 à 9 pour la production d'un médicament destiné au traitement ou à la prévention de maladies ophtalmiques, en particulier de maladies inflammatoires ophtalmiques.

**12.** Utilisation de la composition de conservateur suivant la revendication 10 pour la production d'un médicament destiné au traitement ou à la prévention de maladies ophtalmiques, en particulier de maladies inflammatoires ophtalmiques.

**13.** Procédé de préparation d'une formulation ophtalmique liquide de MAINS, qui consiste à mélanger 0,001% à 10,0% en poids/volume d'un MAINS, 0,001% à 1,0% en poids/volume de chlorure de benzalkonium,

0,001% à 1,0% en poids/volume d'Octoxynol 40, et
de l'eau purifiée : q.s. pour 100%.

14. Procédé suivant la revendication 13, qui consiste en outre à mélanger
0,01% à 1,0% en poids/volume d'un agent chélatant,
une quantité suffisante d'un agent accroissant la tonicité pour parvenir à l'isotonicité avec le liquide lacrymal, et
une quantité suffisante de NaOH 1H ou HCl 1H pour l'ajustement du pH à une valeur de 6,0 à 8,0.

**Patentansprüche**

1. Flüssige ophthalmische Formulierung eines nichtsteroidalen Antiphlogistikums (NSA), umfassend ein NSA in einer zur ophthalmischen Behandlung wirksamen Menge, Benzalkoniumchlorid, eine stabilisierende Menge von Octoxynol 40 und einen wäßrigen Träger.

2. Ophthalmische NSA-Formulierung nach Anspruch 1, welche Dinatrium-EDTA enthält.

3. Ophthalmische NSA-Formulierung nach einem der Ansprüche 1 und 2, worin das NSA ausgewählt ist unter Ketorolac, Indomethacin, Flurbiprofen und Diclofenac, deren Isomeren, pharmazeutisch verträglichen Salzen oder Estern.

4. Ophthalmische NSA-Formulierung nach einem der Ansprüche 1 bis 3, worin das NSA Ketorolac-Tromethamin ist.

5. Ophthalmische NSA-Formulierung nach einem der Ansprüche 1 bis 3, worin das NSA das (l)-Isomer von Ketorolac oder eines seiner pharmazeutisch verträglichen Salze ist.

6. Ophthalmische NSA-Formulierung nach einem der Ansprüche 1 bis 5, umfassend:

| NSA | 0,001 bis 10,0 7 Gew./Vol. |
|---|---|
| Benzalkoniumchlorid | 0,001 bis 1,0 96 Gew./Vol. |
| Octoxynol 40 | 0,001 bis 1,0 96 Gew./Vol. und |
| reines Wasser | q.s. ad 100 %. |

7. Ophthalmische NSA-Formulierung nach Anspruch 6, enthaltend:

| Chelatbildner | 0,01 bis 1,0 % Gew./Vol. |
|---|---|
| Tonisierendes Mittel | q.s., um Isotonizität mit der Tränenflüssigkeit einzustellen, |
| 1N NaOH or 1N HCl | q.s., um den pH auf 6,0 bis 8,0 einzustellen. |

8. Ophthalmische NSA-Formulierung nach Anspruch 7, umfassend:

| NSA | 0,50 % Gew./Vol |
|---|---|
| Benzalkoniumchlorid (50% wäßrige Lösung) | 0,02 % Gew./Vol. |
| Octoxynol 40 (70% wäßrige Lösung) | 0,01 % Gew./Vol. |
| EDTA-Na$_2$ | 0,10 % Gew./Vol. |
| NaCl | 0,79 % Gew./Vol. |
| 1N NaOH oder 1N HCl | q.s, um den pH auf 7,4 ± 0,4 einzustellen; und |
| reines Wasser | q.s ad 100 %. |

9. Ophthalmische NSA-Formulierung nach Anspruch 8, worin das NSA Ketorolac-Tromethamin ist.

**10.** Antimikrobiell wirksames Konservierungssystem für ophthalmologisch verträgliche, Carboxylgruppen-haltige Arzneistoffe, welches Benzalkoniumchlorid und eine stabilisierende Menge von Octoxynol 40 umfaßt.

**11.** Verwendung einer Formulierung nach einem der Ansprüche 1 bis 9 für die Herstellung eines Medikaments zur Behandlung oder Verhütung von Augenkrankheiten, insbesondere Entzündungskrankheiten der Augen.

**12.** Verwendung des Konservierungssystems nach Anspruch 10 für die Herstellung eines Medikaments zur Behandlung oder Verhütung von Augenkrankheiten, insbesondere Entzündungskrankheiten der Augen.

**13.** Verfahren zur Herstellung einer flüssigen ophthalmischen NSA-Formulierung, welches umfaßt das Mischen von
0,001 bis 10,0 % Gew./Vol. NSA,
0,001 bis 1,0 % Gew./Vol. Benzalkoniumchlorid,
0,001 bis 1,0 % Gew./Vol. Octoxynol 40 und
reinem Wasser q.s. ad 100 %.

**14.** Verfahren nach Anspruch 13, welches zusätzlich umfaßt das Zumischen von
0.01 bis 1,0 % Gew./Vol. eines Chelatbildners,
q.s eines tonisierenden Mittels, um Isotonizität mit der Tränenflüssigkeit einzustellen, und
q.s. 1N NaOH oder 1N HCl, um den pH auf 6,0 bis 8,0 einzustellen.

14